(19) Europäisches Patentamt
European Patent Office
Office européen des brevets

(11) EP 3 595 621 B1

(12) EUROPEAN PATENT SPECIFICATION

(45) Date of publication and mention of the grant of the patent:
**27.05.2026 Bulletin 2026/22**

(21) Application number: **18706709.5**

(22) Date of filing: **19.02.2018**

(51) International Patent Classification (IPC):
*A61K 8/34* (2006.01) *A61K 8/36* (2006.01)
*A61K 8/41* (2006.01) *A61Q 5/00* (2006.01)
*A61Q 5/02* (2006.01) *A61Q 5/10* (2006.01)
*A61Q 17/00* (2006.01) *A61Q 19/10* (2006.01)
*A61K 8/92* (2006.01) *A61Q 5/12* (2006.01)
*A61P 17/10* (2006.01) *A61P 31/02* (2006.01)
*A61K 31/045* (2006.01) *A61K 31/05* (2006.01)
*A61K 31/133* (2006.01) *A61K 31/201* (2006.01)
*A61P 31/10* (2006.01)

(52) Cooperative Patent Classification (CPC):
**A61K 8/34; A61K 8/347; A61K 8/361; A61K 8/41; A61K 8/922; A61P 17/10; A61P 31/02; A61P 31/10; A61Q 5/006; A61Q 5/02; A61Q 5/12; A61Q 17/005; A61Q 19/10**

(86) International application number:
**PCT/EP2018/054025**

(87) International publication number:
**WO 2018/166758 (20.09.2018 Gazette 2018/38)**

(54) **ANTIMICROBIAL COMPOSITION COMPRISING THYMOL OR TERPINEOL AND AN ANTIMICROBIAL LIPID**

ANTIMIKROBIELLE ZUSAMMENSETZUNG AUS THYMOL ODER TERPINEOL UND EINEM ANTIMIKROBIELLEN LIPID

COMPOSITION ANTIMICROBIENNE COMPRENANT DU THYMOL OU DU TERPINÉOL ET UN LIPIDE ANTIMICROBIEN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.03.2017 PCT/CN2017/076923**
**26.04.2017 EP 17168120**

(43) Date of publication of application:
**22.01.2020 Bulletin 2020/04**

(73) Proprietors:
- **Unilever Global IP Limited**
**Wirral, Merseyside CH62 4ZD (GB)**
Designated Contracting States:
**CY DE GB IE IT MT**
- **Unilever IP Holdings B.V.**
**6708 WH Wageningen (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
- **CHU, Chung-Ching**
**Shanghai 200335 (CN)**
- **PU, Mingming**
**Shanghai 200335 (CN)**

(74) Representative: **Unilever Patent Group**
**Bronland 14**
**6708 WH Wageningen (NL)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
WO-A1-2013/083393 WO-A2-2013/064360
JP-A- 2010 138 122

- S. SHAPIRO: "The inhibitory action of fatty acids on oral bacteria", ORAL MICROBIOLOGY AND IMMUNOLOGY., vol. 11, no. 5, 1 October 1996 (1996-10-01), DK, pages 350 - 355, XP055239452, ISSN: 0902-0055, DOI: 10.1111/j.1399-302X.1996.tb00193.x
- JOANNE NIKITAKIS, BETH LANGE: "International Cosmetic Ingredient Dictionary and Handbook", vol. 1, 2016, PERSONAL CARE PRODUCTS COUNCIL, article "o-Cymen-5-ol", pages: 967, XP002772423

## Description

### Field of the invention

This invention relates to an antimicrobial composition. The invention more particularly relates to a personal care composition e.g. that for care of hair, body, hand or face which provides anti-microbial efficacy. It more particularly relates to a cleansing/ care composition comprising actives that interact to provide synergistic antimicrobial efficacy for preventing or alleviating the symptoms of problems like acne or dandruff

### Background of the invention

The invention relates to an anti-microbial composition useful for cleaning and/or care of any body part but especially suitable for hair, and scalp care, hand hygiene or for facial care and cleansing.

Acne, also known as Acne vulgaris, is a common skin condition that affects nearly all adolescents and/or adults at some times in their lives. It has a complex etiology, involving abnormal keratinization, excess sebum production, androgen function, bacterial growth, and immune hypersensitivity. Although one or more of the above processes is correlated with acne, the one triggering factor and the exact sequence of events leading to the formation of acne lesions is not been fully understood. Other factors which have been linked to acne are presence of free radicals with subsequent oxidative stress leading to cellular damage. It has been observed that acne usually occurs in areas rich in sebaceous glands like the face, neck and back. A bacterium *Propionibacterium acne* (*P. acnes*) has also been implicated in occurrence of acne.

Acne has been treated in many ways. Most treatments take several weeks to months before a noticeable change is seen. Benzoyl peroxide which has an antibacterial effect has been used for mild cases of comedones and is also believed to prevent formation of other comedones. In very severe cases of acne, antibiotics like tetracycline, erythromycin and clindamycin have been used. Antibiotics are believed to work by several mechanisms, the most important being the decrease in the number of bacteria in and around the follicle. They are also thought to reduce the irritating chemicals produced by the white blood cells in the sebum, thereby reducing the inflammatory response.

Handwashing with antimicrobial soaps, especially after use of the toilet and/ or before partaking of food has been identified as one of the most effective ways of improving human mortality, morbidity and for improving the general quality of life. Sanitizing and disinfecting soap compositions comprising chlorine-based antimicrobial agent such as triclosan are known. If the user, uses a soap where the antimicrobial activity is low or slow, he is likely to have skin with relatively inadequate bacterial removal and may cause contamination of further animate and/or inanimate surfaces and lead to spreading of pathogens and consequent diseases. Hence providing hand and body wash compositions with enhanced antimicrobial activity is paramount for providing enhanced health and quality of life of consumers.

Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter.

Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the *Malassezia* yeasts. To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g. zinc pyrithione (ZPTO), Octopirox® (piroctone olamine), azole antimicrobials (e.g. climbazole), selenium sulfide and combinations thereof.

Most of the treatments to solve the above problems, involve use of synthetic chemicals. Many people do not prefer use of synthetic chemicals since they are believed to be harsh on the human body. Some people believe that synthetic chemicals cause side effects. Hence, more and more people prefer use of materials which are "natural" e.g. actives based on herbal origin.

While the abovementioned problems are mitigated to a large extent through use of compositions containing known antimicrobial actives, there is a need for enhancing the efficacy of these actives while ensuring use of actives derived from natural sources. The present inventors have found that synergistic antifungal as well as antibacterial efficacy can be obtained when certain antimicrobial lipids (AML) are combined with select essential oil actives. The antimicrobial lipids found to be especially useful in the present invention are sapienic acid, palmitoleic acid, sphingosine, dihydrosphingosine, 6-hydroxysphingosine and phytosphingosine. The essential oil actives found to be effective in combination with the above AMLs are terpineol or thymol.

CN104800123 A (Walch Guangzhou Commodity Co Ltd, 2015) discloses an antibacterial soap-based shower gel and a preparation method thereof. The shower gel comprises the following ingredients: thymol, salicylic acid, plants extracts, ethyl alcohol, propylene glycol, erythritol, lauric acid, myristic acid, palmitic acid, potassium hydroxide, glycol

distearate, dihexyl sodium sulfosuccinate, cocamidopropyl betaine, sodium cocoyl glycinate, polyquaternium-7, polyglyceryl fatty acid ester composition, ethylene diamine tetraacetic acid tetrasodium, sodium chloride, citric acid, essence, a preservative and water. The antibacterial soap-based shower gel is soft and friendly to skin, can effectively resist bacteria and moisturize skin and is particularly applicable to people with thicker cuticle, high grease secretion, and skin pruritus.

WO13064360 A2 (Unilever) discloses liquid personal cleaning compositions containing di- or tricarboxylic acid or salts thereof in combination with thymol or terpineol. The combination provides fast-acting synergistic antimicrobial effect.

WO13083393 A1 (Unilever) discloses a method of disinfecting a surface and an antibacterial composition which does so. The compositions comprise select antibacterial actives, specific polymers and selected hydrotropes.

To our knowledge, the AMLs claimed in the present invention in combination with the selected essential oil actives have not been disclosed for synergistic antimicrobial activity.

It is thus an object of the present invention to provide for a personal care composition that exhibits synergistic antimicrobial activity as compared to the individual components.

## Summary of the invention

According to the first aspect of the present invention there is an anti-microbial composition comprising:

(i) 0.01 to 5% by weight of an essential oil active chosen from thymol or terpineol;
(ii) 0.01 to 5% by weight of an antimicrobial lipid selected from sapienic acid, palmitoleic acid, sphingosine, dihydrosphingosine, phytosphingosine, and 6-hydroxysphingosine; and,
(iii) a cosmetically acceptable vehicle.

The second aspect of the present invention relates to a non-therapeutic method of providing antimicrobial efficacy to a topical surface of a person comprising the step of applying a composition of the first aspect on to the desired surface.

In accordance with another aspect is disclosed a therapeutic method of providing antimicrobial efficacy to a topical surface of a person comprising the step of applying a composition of the first aspect on to the desired surface.

In accordance with one more aspect is disclosed an anti-microbial composition comprising:

(i) 0.01 to 5% by weight of an essential oil active chosen from thymol or terpineol;
(ii) 0.01 to 5% by weight of an antimicrobial lipid selected from sapienic acid, palmitoleic acid, sphingosine, dihydrosphingosine, phytosphingosine, and 6-hydroxysphingosine; and,
(iii) a cosmetically acceptable vehicle, for use to treat acne or dandruff.

## Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'an antimicrobial composition' as used herein, is meant to include a composition for topical application to skin, hair and/or scalp of mammals, especially humans. Such a composition is generally applied on to the desired topical surface of the body for a few seconds to up to 24 hours. When the time of application is low say of the order of a few seconds to a few minutes after which the composition is rinsed off with water or wiped away, such a composition is known as a

cleansing composition or a wash-off composition. When the composition is applied for longer, say from several minutes to up to 24 hours and washed off usually during the process of normal personal cleaning, such a composition is known as a leave-on composition. The composition as per the present invention may be of the wash-off or of the leave-on type. Of the two, it is preferably of the wash-off type. It includes any product applied to a human body for also improving appearance, cleansing, odor control or general aesthetics. The composition of the present invention can be in the form of a liquid, lotion, cream, foam, scrub, gel, bar, shampoo, conditioner, handwash, facewash or bodywash product. It is more preferably used for preventing or alleviating the symptoms of dandruff on the scalp and/or hair, for antiacne benefit, or for disinfecting the hand or other parts of the human body.

The present invention more particularly relates to an antimicrobial cleansing composition.

The antimicrobial lipids which in combination with the essential oil actives which provide the synergistic antimicrobial activity include sapienic acid, sphingosine, dihydrosphingosine, palmitoleic acid, phytosphingosine, 6-hydroxysphingosine or combinations of one of more of the AMLs. More preferred AMLs are sapienic acid, phytosphingosine and dihydrosphingosine most preferred being phytosphingosine and dihydrosphingosine.

Sapienic acid and palmitoleic acid are generally added into the antimicrobial agents in purified form. These fatty acids may be chemically synthesized or naturally sourced and are in free fatty acid form. Natural substances which contain these fatty acids and its derivatives may come from animal or plant sources, including but not limited to plant seed extract, marine oils, animal oil and microbial ferments. Specifically, sapienic acid was reported as a major constituent of the seed oil of *Thunbergia alata* (85% by weight as the triglyceride). Purified sapienic acid or seed oil extract comprising sapienic acid may be added to the
composition, while ensuring that sapienic acid is present in the desired concentration in the composition of the present invention.

Sphingosine, phytosphingosine, dihydrosphingosine, and 6-hydroxy sphingosine are sphingoid bases present on the human skin. They can be synthesized chemically or produced via suitable biotechnological process that lead to formation of sphingoid bases by yeast from materials like sugar. Sphingoid bases may be added to the antimicrobial composition in purified form. Alternatively, fermentation broth consisting of high quantities of sphingoid bases may be used, while ensuring that sphingoid bases are present in the desired concentration in the composition of the present invention. The AMLs are included in 0.01 to 5%, preferably 0.1 to 2% by weight of the composition.

The essential oil actives that are efficacious in combination with the AMLs in the present invention are thymol or terpineol.

Thymol may be added to the antimicrobial composition in purified form. Alternatively, thyme oil or thyme extract comprising thymol may be added to the antimicrobial composition, while ensuring that thymol is present in the desired concentration in the composition of the present invention. Thyme oil or thyme extract is obtained from the thyme plant. Thyme plant refers to a plant belonging be genus *Thymus* and includes but is not limited to the following species: *Thymus vulgaris, Thymus zygis, Thymus satureoides, Thymus mastichina, Thymus broussonetti, Thymus maroccanus, Thymus pallidus, Thymus algeriensis, Thymus serpyllum, Thymus pulegoide,* and *Thymus citriodorus.*

The terpineol is preferably selected from alpha-terpineol, beta-terpineol, gamma-terpineol or mixtures thereof. It is particularly preferred that the terpineol is alpha-terpineol. Terpineol may be added to the antimicrobial composition in purified form. Alternatively, pine oil comprising terpineol may be added to the antimicrobial composition.

The antimicrobial composition comprises 0.01 to 5% of the essential oil actives listed above, preferably from 0.05 to 2.0%, more preferably 0.1 to 1.0% by weight of the composition of the present invention.

As per an especially preferred aspect of the invention, the composition comprises a mixture of thymol and terpineol. In this preferred aspect, thymol is included in 0.01 to 2% preferably 0.05 to 1% and terpineol in 0.01 to 5%, preferably 0.1 to 2% by weight of the composition.

It is preferred that the weight ratio of the essential oil active to the antimicrobial lipid is in the range of 1:1 to 3000:1 at the cellular level for the antimicrobial action to be most effective.

Evonik Industries is a major supplier of AMLs including sphingosine, phytosphingosine, cholesterol, and fatty acids.

Without wishing to be bound by theory, the inventors believe that the synergistic anti-microbial efficacy of the essential oil actives in combination with AML is through microbial membrane disruption.

In the present invention, it is preferred that antimicrobial alcohols (low molecular weight alcohols having one to 7 carbon atoms) are substantially absent. By substantially absent is meant that the concentration of the alcohol is less than an amount that is necessary for antimicrobial therapeutic activity. Preferably, the antimicrobial alcohol is present in less than 1%, more preferably less than 0.1% and optimally absent from the invention.

The composition of the invention comprises a cosmetically acceptable vehicle. According to one aspect the cosmetically acceptable carrier comprises water. According to another preferred aspect, the carrier additionally comprises a surfactant. The cosmetically acceptable vehicle is such that the composition can be prepared as a is a cream, lotion, gel, powder, ointment, body wash, hand wash or face wash product, shampoo, hair conditioner, or a soap bar preferably as a shampoo, conditioner, body wash, hand wash or face wash product.

Skin Care

The composition of the invention may be used for skin care. The cosmetically acceptable base in such cases may be a liquid or solid material. Typically, base is present in an amount ranging from 10 to 99.9%, more preferably from 20 to 95%, most preferably from 40 to 85% by total weight of the composition including all ranges subsumed therein. It is particularly preferred that the cosmetically acceptable carrier includes water. Water is preferably included in an amount from 30 to 90%, more preferably from 30 to 85%, most preferably from 30 to 80% by total weight of the sunscreen composition. Besides water, suitable carrier classes include silicones, polyhydric alcohols, hydrocarbons, triglycerides and thickening powders.

The skin care composition of the invention may be in any form including toners, lotions, creams, mousses, scrub, serum or gel that is suitable for topical application to the skin. The composition can be either a leave-on product such as skin lotions, creams, antiperspirants, deodorants, lipsticks, foundations, mascara, sunless tanners and sunscreen lotions or a rinse-off product such as shower gels and toilet bars. It is preferred that the composition is a skin lotion or a cream.

The composition may comprise an emollient oil that act as a co-solvent. Suitable emollient oils include, for example, ester of alkoxylated aromatic alcohol with fatty carboxylic acid, esters of polyglycols or diols with fatty carboxylic acid such as caprylic/capric triglyceride, ester of fatty alcohol and fatty acid, alkoxylated derivative of benzyl alcohol and mixtures thereof. Preferably the emollient oil is caprylic/capric triglyceride.

Typically, such compositions comprise co-solvent in an amount from 0.01 to 10%, more preferably from 0.1 to 8%, most preferably from 1 to 6%, based on the total weight of the sunscreen composition and including all ranges subsumed therein.

The composition may additionally comprise sunscreen agents such as inorganic sunscreens. For example, zinc oxide, titanium dioxide, iron oxide, silica such as fumed silica. The amount of such sunscreen agents is preferably incorporated from 0.1 to 5% by total weight of the sunscreen composition.

The composition of the invention may comprise a UV-A sunscreen agent selected from the group consisting of a dibenzoylmethane derivative, a triazine derivative, a benzophenone derivative and mixtures thereof. In a preferred embodiment, the UV-A sunscreen agent comprises or is a dibenzoylmethane derivative, for example, butyl methoxydibenzoylmethane (sold under the tradename Parsol 1789).

Typically, the sunscreen composition of the present invention comprises from 0.1 to 15% by weight of the UV-A sunscreen agent, more preferably from 0.1 to 10%, most preferably from 1 to 5%, based on the total weight of the composition and including all ranges subsumed therein.

The composition of the invention may also comprise a UV-B sunscreen agent. Suitable UV-B sunscreen agent of the invention is selected from the group consisting of a benzophenone, an anthranilate, a salicylate, a cinnamate, a camphor, benzylidene malonate, a triazone, and derivatives thereof. In a preferred embodiment, the UV-B sunscreen agent comprises or is a cinnamate derivative, for example, ethylhexyl methoxycinnamate (sold under the trade name Parsol MCX).

Typically, the composition comprises from 0.1 to 20% by weight of the UV-B sunscreen agent, more preferably from 0.5 to 18%, most preferably from 1 to 15%, based on the total weight of the composition and including all ranges subsumed therein.

A skin lightening agent may also be incorporated into the composition of the invention. Most preferred skin lightening active is a Vitamin B3 compound. Vitamin B3 compound maybe nicacin, nicotinic acid or niacinamide, preferably niacinamide.

Niacinamide is known for secretion of AMPs from keratinocytes. The AMPs thus secreted provides for improving the immunity of the external surface of the body e.g. on the scalp. Thus with the use of niacinamide in the composition of the invention the anti-dandruff efficacy is expected to be enhanced not just through anti-fungal activity of the composition of the invention but by providing a boost to the scalp's own protection shield against germs, through use of niacinamide. It is expected that this combination could provide further long-lasting protection e.g. up to 24 hours of protection against germs.

Niacinamide is preferably present in 0.01 to 5%, more preferably 0.1 to 3% by weight of the composition. Suitable skin lightening agents other than Vitamin B3 and its derivatives are kojic acid, arbutin, tranexamic acid, placental extract, ascorbic acid and its derivatives (e.g. magnesium ascorbyl phosphate, sodium ascorbyl phosphate, ascorbyl glucoside, and ascorbyl tetraisopalmitates), aloe extract, ammonium lactate, azelaic acid, citrate esters, ellagic acid, glycolic acid, green tea extract, hydroquinone, lemon extract, linoleic acid, vitamins like vitamin B6, vitamin B12, vitamin C, vitamin A, a dicarboxylic acid, resorcinol derivatives, hydroxycarboxylic acid like lactic acid and their salts (e.g. sodium lactate) or a mixture thereof. Typically, the skin lightening agent is present in an amount from 0.1 to 10%, more preferably from 0.2 to 5%, most preferably from 0.3 to 3% by total weight of the composition including all ranges subsumed therein.

Skin care compositions may also comprise other ingredients which are common in the art to enhance physical properties and performance. Suitable ingredients include but are not limited to humectants, thickeners, opacifiers, binders, colorants and pigments, pH adjusting agents, preservatives, optics, perfumes, viscosity modifiers, biological additives, buffering agents, conditioners, natural extracts, essential oils and skin benefit agents including anti-inflammatory agents, cooling agents, antiperspirant agents, anti-aging agents, anti-acne agents, anti-microbial agents and antioxidants.

Skin cleansing

The composition of the invention may be used for skin care e.g. body or face wash. The antimicrobial composition may further comprise a surfactant. The preferred surfactants are nonionic surfactants, such as $C_8$-$C_{22}$, preferably $C_8$-$C_{16}$ fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Thus, in a highly preferred aspect, the antimicrobial compositions include the surfactant selected from the group of anionic surfactants, fatty acid amide, alkyl sulphate, linear alkyl benzene sulphonate, and combinations thereof.

When the surfactants are present, the antimicrobial composition preferably comprises 1 to 90% surfactant by weight of the composition

When surfactant is used, a particularly preferred surfactant is soap. Soap is a suitable surfactant for personal washing applications of the antimicrobial composition of the invention. The soap is preferably $C_8$-$C_{24}$ soap, more preferably $C_{10}$-$C_{20}$ soap and most preferably $C_{12}$-$C_{18}$ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also be used in other desired proportions.

When present, the soap, of the present is preferably present in an amount of 1 to 90%, preferably from 10 to 85%, more preferably 25 to 75% by weight of the composition. Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, further more preferably at least 5% by weight of the composition. When water is the carrier, a preferred liquid composition comprises 10 to 99.8% by weight water. The liquid antimicrobial composition is useful as a skin antiseptic liquid, for skin cleansing, in particular for hand wash or a face wash. When water is the carrier, a preferred solid composition comprises 5 to 30% by weight water. The solid antimicrobial composition is preferably in form of a shaped solid, more preferably a bar. The solid antimicrobial composition is particularly useful for skin cleansing in particular for hand wash or a face wash.

According to another aspect, inorganic particulate material is also a suitable carrier. When inorganic particulate material is the carrier, the antimicrobial composition is in a solid form. Preferably the inorganic particulate material is talc. When the inorganic particulate material is talc, the solid antimicrobial composition is particularly useful as a talcum powder for application on face or body.

In another aspect of the present invention, the composition of the present invention is suitable for use in wipes for personal hygiene.

Shampoo

As per an especially preferred aspect of the invention, the composition is a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, furthermore preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: $RO(CH_2CH_2O)_nSO_3M$; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

Examples of further suitable anionic cleansing surfactants are the alkaryl sulphonates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl ether sulphosuccinate, sodium dodecylbenzene sulphonate, triethanolamine dodecylbenzene sulphonate, lauryl ether carboxylic acid and sodium N-lauryl sarcosinate.

Suitable preferred additional anionic cleansing surfactants are sodium lauryl ether sulphosuccinate(n)EO, (where n is from 1 to 3), lauryl ether carboxylic acid (n) EO (where n is from 10 to 20).

Mixtures of any of the foregoing anionic cleansing surfactants may also be suitable.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of a shampoo composition of the invention is preferably equal to or higher than 4.0, more preferably in the range of 5.0 to 7.0.

The composition as per the invention especially for anti-dandruff shampoos preferably additionally comprises one or more antidandruff agent. Preferred agents are selected form zinc pyrithione, azole compounds (e.g. climbazole or ketoconazole), octopirox, selenium sulfide or combinations thereof. As per the present invention it is desirable to include zinc pyrithione as the antidandruff agent.

Zinc pyrithione (ZPTO) is shorthand for zinc 1-hydroxy-2-pyridinethione.

The polyvalent metal salt of pyrithione is represented by the following general formula:

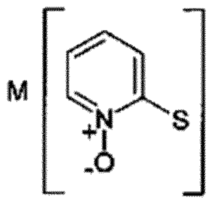

In the case of zinc pyrithione, M, the metal cation is zinc.

Zinc pyrithione is preferably present in 0.1 to 3.0%, more preferably from 0.1 to 2.0% based on weight of the composition. ZPTO is a particulate material. While the particle size is not critical to achieve the benefits of the present invention, the particle size of ZPTO is preferably from 0.25 to 8 micrometer, more preferably from 0.5 to 8.0 micrometer, and further more preferably from 1.0 to 7.5 micrometer. ZPTO is commercially available from Kolon Life Science Inc., Sino Lion (USA) Ltd, Lonza, and other suppliers. The presence of an additional zinc compound in the composition is believed to improve the antidandruff efficacy of the metal salt of pyrithione. Suitable zinc compounds are zinc oxide, zinc citrate, zinc malonate, zinc carbonate or combinations thereof. The zinc compound is preferably present in 0.1 to 3%, more preferably 0.1 to 1.5% by weight of the composition.

Shampoo composition as per the invention preferably additionally comprises a conazole fungicide. Preferably the conazole fungicide is selected form ketoconazole, climbazole or mixtures thereof. The azole fungicide is preferably included in 0.01 to 2%, more preferably 0.025 to 0.75% by weight of the composition. The presence of a conazole fungicide is believed to improve the deposition of zinc pyrithione.

The composition preferably additionally comprises a vitamin B3 compound. The preferred vitamin B3 compound is niacinamide.

Niacinamide is preferably present in 0.1 to 5%, more preferably 0.5 to 5%, further more preferably 0.5 to 3%, and optimally 1.0 to 3.0% by weight of the composition.

Suspending Agent

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan®.

Mixtures of any of the above suspending agents may be used. Preferred is a mixture of cross-linked polymer of acrylic acid and crystalline long chain acyl derivative.

Suspending agent, if included, will generally be present in a shampoo composition of the invention at levels of from 0.1 to 10%, preferably from 0.5 to 6%, more preferably from 0.5 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The composition of the invention is preferably aqueous based. It preferably comprises high amounts of water preferably from 70 to 95% by weight of the composition.

Hair Conditioner

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of a conditioner composition of the invention is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful

cationic surfactant for use in conditioners according to the invention is behenyltrimethylammonium chloride, available commercially, for example as GENAMIN KDMP, ex Clariant. Yet another preferred cationic surfactant is stearamidopropyl dimethylamine.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

The invention also relates to a method of providing antimicrobial efficacy to a topical surface of a person comprising the step of applying a composition of the invention on to the desired surface.

The method may be utilized for preventing or treating acne, dandruff or for maintaining general hygiene.

The present invention also relates to a method of inhibiting the growth of bacteria or fungus from the surface of the body. The method is especially effective against fungus like *Malassezia furfur* and bacteria like *P. acnes.* The method comprises the step of applying a composition of the first aspect on to the scalp or any other body surface of an individual. Alternatively, and preferably, the above step is followed by rinsing the surface with water. The step of rinsing is generally carried out within 1 to 5 minutes after application of the composition on the skin or hair/ scalp. According to one aspect, the invention provides for non-therapeutic benefits.

The invention will now be illustrated with reference to the following non-limiting examples.

## Examples

Examples 1 to 8:

Synergistic interaction between the essential oil active and the AML compounds was determined using the ΣFIC assay against *P. acnes.* A brief description of the method is given below.

Methods: ∑FIC assay against *P. acnes*

Microorganism preparation:

*P. acnes* (ATCC 6919) is inoculated on Reinforced Clostridial Medium Agar plate (RCMA, solution A) and cultured anaerobically in an anaerobic jar at 37 °C for 72 to 96 hours. A single colony was cultured into Reinforced Clostridial Medium (RCM, solution B) and incubated anaerobically at 37°C for 72 to 96 hours. Final cell density is adjusted to around $5\times10^7$ cells/ml by diluting using RCM. Then the inoculum is further diluted 1:1000 into Luria-Bertani medium (LB, solution C) to get around $5*10^4$ cell/ml.

Solution A

Reinforced Clostridial Medium Agar (RCMA)
38 g RCM (HopeBio)
15 g Agar (Oxoid)
Deionized Water to 1000 ml
Autoclave

Solution B

Reinforced Clostridial Medium (RCM)
38 g RCM (HopeBio)
Deionized Water to 1000 ml
Autoclave

Solution C

Luria-Bertani (LB)
10 g Tryptone (Oxoid)
10 g Sodium chloride (SCR)
5 g Yeast Extract (Oxoid)
Deionized Water to 1000 ml
Autoclave

In-vitro susceptibility testing

Essential oil active was serially diluted (2-fold) to prepare in a range of 10 to 10,000 ppm in the LB growth medium. The AML compound was serially diluted (2-fold) to prepare in a range of 15.6 to 1000 ppm for sapienic acid, and palmitoleic acid; and 2-125 ppm for other AMLs in the LB growth medium.

Binary combinations of essential oil and AML compound were prepared in 96-well plate by mixing 20 μl of essential oil solution with 20 μl of AML compound. The solution in each well were further mixed with 160 μl of *P. acnes* in LB. The final cell density in the testing plate is around $4*10^4$ cell/ml, and the final concentration of actives in each well of the 96-wel plate were shown below.

Essential oil active concentrations (in ppm): 1000, 500, 250, 125, 62.5, 31.3, 15.6, 7.8, 3.9, 2.0, 1.0 and 0.

The fatty acid AML sapienic acid and palmitoleic acid compound concentrations (in ppm): 100, 50, 25, 12.5, 6.25, 3.13, 1.56, and 0. The sphingosine AML compound concentrations (in ppm): 12.5, 6.3, 3.1, 1.6, 0.8, 0.4, 0.2 and 0.

The plate was incubated anaerobically at 37°C for 50 hours. Then 20 μl of alamar blue (0.01%) was added into each well with a further incubation aerobically for two to three days to observe the color change. Red color indicates microbial growth and the non-changing blue color indicates no growth. In addition, it was also confirmed that the testing compound alone will not cause color change of alamar blue with a control mixture without adding microbes.

Calculations:

Minimum Inhibition Concentration (MIC):

The MIC is defined as the absolute lowest concentration of active that provides complete microbial growth inhibition as indicated by the blue color of alamar blue under the tested condition.

The MIC of sapienic acid, palmitoleic acid, sphingosine, phytosphingosine and dihydrosphingosine was determined to be 1 to 4 ppm, 1 ppm, 0.4 ppm, 0.2 ppm, and 0.2 ppm respectively. The MIC of thymol is 62.5 ppm and that of terpineol is 1000 ppm.

Fractional Inhibition Concentration (FIC):

The differing behaviour of inhibitory antimicrobials in isolation and mixtures have been widely explored using the concept of the Fractional Concentration (FC) and Fractional Inhibitory Concentration (FIC). The parameter can be defined as follows:

$$\text{FIC (component a)} = \frac{\text{MIC (component a tested in the mixture)}}{\text{MIC (component a tested as a single active)}}$$

Synergy and Additivity

The interactions between antimicrobials can be additive, synergistic or antagonistic depending on whether the efficacy of the combination is equivalent to, greater than or less than that obtained for the same total concentration of the individual components when tested alone.

These relationships can be expressed mathematically by summing the fractional MIC values for all the components present in the mixture to give the "fractional inhibition index". There is no consistent approach in the academic or patent literature in defining precise limiting ΣFIC values that differentiate synergy from additivity or antagonism. In this study, we have adopted a liberal approach defining any binary mixture with ΣFIC < 0.9 as showing evidence of synergistic behavior.

$$\Sigma FIC = FIC\ (component\ 1) + FIC\ (component\ 2)$$

$$\Sigma FIC = 1 \text{ corresponds to additive microbial activity}$$

$$\Sigma FIC > 1 \text{ corresponds to antagonistic microbial activity}$$

$$\Sigma FIC < 0.9 \text{ corresponds to synergistic microbial activity}$$

Results:

Based on the concentration range of the essential oil actives (0 to 1000 ppm) and the AML compound (0 to 100 ppm) at which the experiments were carried out, the ΣFIC of the combinations is given below in Table - 1.

Table - 1

| Example | Combination | ΣFIC |
|---|---|---|
| 1 | Thymol + sapienic acid | 0.52 |
| 2 | Thymol + sphingosine | 0.50 |
| 3 | Thymol + dihydrosphingosine | 0.50 |
| 4 | Thymol + phytosphingosine | 0.50 |
| 5 | Thymol + palmitoleic acid | 0.51 |
| 6 | Terpineol + sapienic acid | 0.56 |
| 7 | Terpineol + sphingosine | 0.75 |
| 8 | Terpineol + dihydrosphingosine | 0.50 |
| 9 | Terpineol + phytosphingosine | 0.56 |
| 10 | Terpineol + palmitoleic acid | 0.625 |

The data in Table above indicates that each of the anti-microbial lipids of the present invention in combination with essential oil actives like thymol and terpineol exhibit synergistic antimicrobial activity.

The experiments described above were conducted in an invitro assay to evaluate the synergistic antimicrobial behaviour. It is expected that the concentrations to be used to prepare a composition for topical use would be vastly different. The concentrations could be orders of magnitude higher due to the following reasons that affect the difference in concentration in the bulk as compared to that at the cellular level. The composition may be formulated as an emulsion or a gel with very many additional ingredients which affect the concentration of the desired actives in the oil phase and in the water phase which could be very different. They may also have very different physical and hydrodynamic properties like partition coefficients, diffusional rates, convective transport rates and rheological properties. Therefore, it is expected that the concentrations to be used when formulated as a composition would be different from that at the cellular level, at which the experiments were carried out, usually orders of magnitude higher.

## Claims

**1.** An anti-microbial composition comprising

(i) 0.01 to 5% by weight of an essential oil active chosen from thymol, or terpineol;
(ii) 0.01 to 5% by weight of an antimicrobial lipid selected from sapienic acid, palmitoleic acid, sphingosine,

dihydrosphingosine, phytosphingosine, and 6-hydroxysphingosine; and,
(iii) a cosmetically acceptable vehicle.

**2.** A composition as claimed in claim 1 wherein the antimicrobial lipid is chosen from sapienic acid, phytosphingosine and dihydrosphingosine.

**3.** A composition as claimed in claim 1 or 2 comprising thymol and terpineol.

**4.** A composition as claimed in any one of the preceding claims wherein the composition is a cream, lotion, gel, powder, ointment; body wash, hand wash or face wash product; shampoo, hair conditioner, or a soap bar.

**5.** A composition as claimed in any one of the preceding claims wherein the cosmetically acceptable vehicle comprises a surfactant.

**6.** A non-therapeutic method of providing antimicrobial efficacy to a topical surface of a person comprising the step of applying a composition as claimed in any one of the preceding claims on to the desired surface.

**7.** An anti-microbial composition comprising:

(i) 0.01 to 5% by weight of an essential oil active chosen from thymol or terpineol;
(ii) 0.01 to 5% by weight of an antimicrobial lipid selected from sapienic acid, palmitoleic acid, sphingosine, dihydrosphingosine, phytosphingosine, and 6-hydroxysphingosine; and,
(iii) a cosmetically acceptable vehicle, for use to treat acne or dandruff.

## Patentansprüche

**1.** Antimikrobielle Zusammensetzung, umfassend

(i) 0,01 bis 5 Gew.-% eines ätherischen Ölwirkstoffs, ausgewählt unter Thymol oder Terpineol;
(ii) 0,01 bis 5 Gew.-% eines antimikrobiellen Lipids, ausgewählt unter Sapiensäure, Palmitoleinsäure, Sphingosin, Dihydrosphingosin, Phytosphingosin und 6-Hydroxysphingosin; und
(iii) einen kosmetisch akzeptablen Träger.

**2.** Zusammensetzung nach Anspruch 1, wobei das antimikrobielle Lipid unter Sapiensäure, Phytosphingosin und Dihydrosphingosin ausgewählt ist.

**3.** Zusammensetzung nach Anspruch 1 oder 2, die Thymol und Terpineol enthält.

**4.** Zusammensetzung nach irgendeinem der vorhergehenden Ansprüche, wobei es sich bei der Zusammensetzung um eine Creme, eine Lotion, ein Gel, ein Puder, eine Salbe, ein Duschgel, ein Handwaschmittel oder ein Gesichtswaschmittel, ein Shampoo, eine Haarspülung oder ein Seifenstück handelt.

**5.** Zusammensetzung nach irgendeinem der vorhergehenden Ansprüche, wobei der kosmetisch akzeptable Träger ein Tensid umfasst.

**6.** Nicht-therapeutisches Verfahren zur Erzielung einer antimikrobiellen Wirksamkeit auf einer topischen Oberfläche einer Person, umfassend den Schritt des Aufbringens einer Zusammensetzung, wie in irgendeinem der vorhergehenden Ansprüche beansprucht, auf die gewünschte Oberfläche.

**7.** Antimikrobielle Zusammensetzung, umfassend:

(i) 0,01 bis 5 Gew.-% eines ätherischen Ölwirkstoffs, ausgewählt unter Thymol oder Terpineol;
(ii) 0,01 bis 5 Gew.-% eines antimikrobiellen Lipids, ausgewählt unter Sapiensäure, Palmitoleinsäure, Sphingosin, Dihydrosphingosin, Phytosphingosin und 6-Hydroxysphingosin; und
(iii) einen kosmetisch akzeptablen Träger,

zur Verwendung bei der Behandlung von Akne oder Schuppen.

## Revendications

**1.** Composition antimicrobienne comprenant

(i) 0,01 à 5 % en poids d'un principe actif d'huile essentielle choisi parmi le thymol ou le terpinéol ;
(ii) 0,01 à 5 % en poids d'un lipide antimicrobien choisi parmi l'acide sapiénique, l'acide palmitoléique, la sphingosine, la dihydrosphingosine, la phytosphingosine et la 6-hydroxysphingosine ; et
(iii) un véhicule acceptable du point de vue cosmétique.

**2.** Composition selon la revendication 1, dans laquelle le lipide antimicrobien est choisi parmi l'acide sapiénique, la phytosphingosine et la dihydrosphingosine.

**3.** Composition selon la revendication 1 ou 2, comprenant du thymol et du terpinéol.

**4.** Composition selon l'une quelconque des revendications précédentes, dans laquelle la composition est une crème, une lotion, un gel, une poudre, une pommade; un produit de type gel douche, savon pour les mains ou nettoyant pour le visage; un shampooing, un après-shampooing ou un pain de savon.

**5.** Composition selon l'une quelconque des revendications précédentes, dans laquelle le véhicule acceptable du point de vue cosmétique comprend un tensioactif.

**6.** Procédé non thérapeutique de fourniture d'une efficacité antimicrobienne à une surface topique d'une personne comprenant l'étape d'application d'une composition selon l'une quelconque des revendications précédentes sur la surface souhaitée.

**7.** Composition antimicrobienne comprenant :

(i) 0,01 à 5 % en poids d'un principe actif d'huile essentielle choisi parmi le thymol ou le terpinéol ;
(ii) 0,01 à 5 % en poids d'un lipide antimicrobien choisi parmi l'acide sapiénique, l'acide palmitoléique, la sphingosine, la dihydrosphingosine, la phytosphingosine et la 6-hydroxysphingosine ; et
(iii) un véhicule acceptable du point de vue cosmétique, destinée à être utilisée pour traiter l'acné ou les pellicules.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104800123 A
- WO 13064360 A2
- WO 13083393 A1